# EUROPEAN PATENT APPLICATION

(11) **EP 2 777 707 A1**
(43) Date of publication of application: **17.09.2014**
(21) Application number: 14158641.2
(22) Date of filing: 10.03.2014
(51) Int. Cl.: A61K 31/7084, A61K 31/7088, A61P 35/00

(54) **Method of Treating Brain Tumors**

(30) Priority: 11.03.2013 US 201361775908 P
(71) Applicant: Wake Forest University Health Sciences, Winston-Salem, NC 27157 (US)
(72) Inventor: Gmeiner, William H., Yadkinville North Carolina 27055 (US); Debinski, Waldemar, Winston-Salem North Carolina 27104 (US)
(74) Representative: Gibbs, Richard

(57) **Abstract**

Provided herein are methods useful for treating a brain tumor in a subject in need thereof, comprising administering to said subject an active agent comprising poly-FdUMP or a pharmaceutically acceptable salt thereof. Also provided are compositions comprising poly-FdUMP and one or more additional active agents useful for treating a brain tumor.

## Description

### STATEMENT OF GOVERNMENT SUPPORT

The present invention was made with government support under Grant Number CA 102532 from the National Institutes of Health. The U.S. Government has certain rights to this invention.

### BACKGROUND

Glioblastoma (GBM) is the most common malignant brain tumor and one of the deadliest of human malignancies (Krex et al., Long-term survival with glioblastoma multiforme. Brain. 2007;130(pt 10):2596-2606; Hulleman et al., Molecular mechanisms in gliomagenesis. Adv Cancer Res 2005;94:1-27). Optimal therapy results in survival times of ∼15 months for newly diagnosed cancer and 5-7 months for recurrent disease (Henriksson et al., Impact of therapy on quality of life, neurocognitive function and their correlates in glioblastoma multiforme: a review. J Neurooncol. 2011;104(3):639-646).

Glioblastoma is very difficult to treat because the tumor cells are very resistant to conventional therapies. Also, the brain is susceptible to damage due to conventional therapy and has a very limited capacity to repair itself. New therapeutic modalities are urgently needed.

The fluoropyrimidine (FP) anti-tumor agent FdUMP[10] (F10) displayed strong anti-leukemic activity towards genetically-engineered syngeneic murine models (Zuber et al., Mouse models of human AML accurately predict chemotherapy response. Genes Dev. 2009;23(7):877-889) of acute myeloid (Pardee et al., Unique dual targeting of thymidylate synthase and topoisomerase 1 by FdUMP[10] results in high efficacy against AML and low toxicity. Blood. 2012; 119(15):3561-3570) and acute lymphoid leukemia (Pardee et al., In Preparation) that replicate the poor response of human patients to chemotherapy. The anti-leukemic activity of F10 was achieved with markedly reduced systemic toxicities relative to the current standard of care (Feldman, Too much ara-C? Not enough daunorubicin? Blood. 2011.117(8):2299-2300) consisting of an anthracycline in combination with cytarabine indicating F10 displays excellent selectivity for malignant cells. F10 also displays cytotoxicity towards the central nervous system (CNS) malignancies included in the NCI 60 cell line panel (Gmeiner et al., Genome-wide mRNA and microRNA profiling of the NCI 60 cell-line screen and comparison of FdUMP[10] with fluorouracil, floxuridine, and topoisomerase 1 poisons. Mol Cancer Ther. 2010;9(12):3105-3114).

### BRIEF SUMMARY OF EMBODIMENTS

Provided herein are methods of treating a brain tumor in a subject in need thereof (e.g., a human subject), comprising administering to said subject an active agent as described herein in an amount effective to treat said tumor. In some embodiments, the active agent comprises poly-FdUMP or a pharmaceutically acceptable salt thereof (e.g., FdUMP[9], FdUMP[10]).

In some embodiments, the subject is administered a single active agent consisting of said poly-FdUMP or pharmaceutically acceptable salt thereof in an amount effective to treat the tumor. In other embodiments, the active agent is administered in combination with one or more other agents and/or therapies.

In some embodiments, the brain tumor is a glioma. In some embodiments, the brain tumor is an astrocytoma. In some embodiments, the brain tumor is glioblastoma multiforme (GBM).

In some embodiments, the active agent is administered to said subject by intra-cerebral administration. In some embodiments, the active agent is administered by intracerebroventricular infusion. In some embodiments, the active agent is administered by intrathecal infusion. In some embodiments, the active agent is administered into the brain of said subject by convection-enhanced delivery (CED). In some embodiments, the active agent is provided as a liposomal formulation.

In some embodiments, the poly-FdUMP is covalently linked at the 3' end thereof to a levulinyl group. In some embodiments, the poly-FdUMP is covalently linked at the 3' end thereof to a hydrophobic molecule (*e.g.*, cholesterol).

Also provided are compositions comprising two or more of the active agents described herein (*e.g*., poly-FdUMP, another thymidylate synthase (TS) inhibitor, an epidermal growth factor receptor (EGFR) inhibitor, an inhibitor of casein kinase 1 or casein kinase 2, and/or temozolomide (TMZ)). In some embodiments the compositions are provided in a liquid carrier (e.g., suitable for infusion into the subject).

A further aspect of the invention is an active agent as described herein for use in carrying out a method of treatment as described herein, and/or for the preparation of a medicament for carrying out a method of treatment as described herein.

### BRIEF DESCRIPTION OF THE DRAWINGS

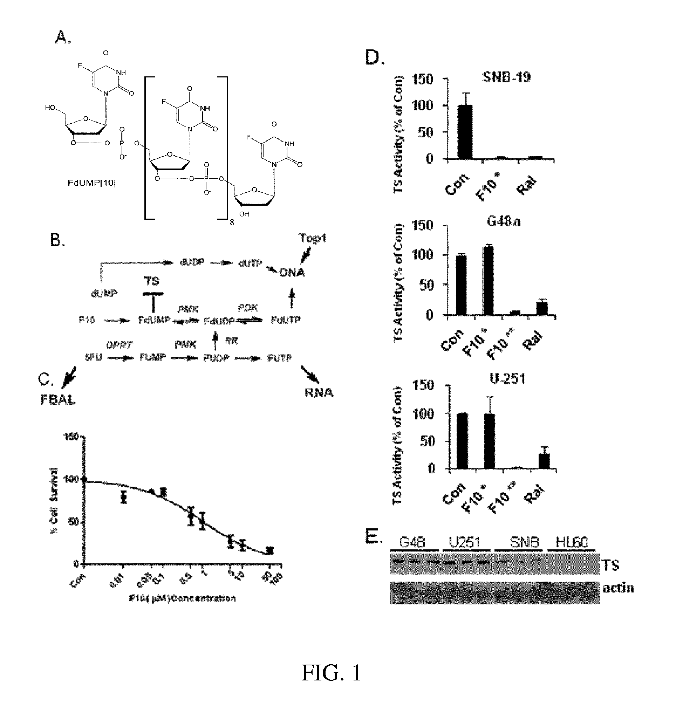

**Figure 1****. F10 is cytotoxic to GBM cells through induction of thymineless death.** (A) Chemical structure of F10; (B) Metabolic conversion of F10 to the thymidylate synthase (TS) inhibitory metabolite FdUMP and FdUTP that is incorporated into DNA and causes Top1-mediated DNA double strand breaks. FBAL and RNA-directed effects cause neurotoxicity and other side effects; (C) Cytotoxicity of F10 to G48 cells; (D) TS inhibitory activity of F10 towards SNB-19 (top), G48a (middle), and U-251 MG (bottom) cells at 10⁻⁶ (*) and 10⁻⁵ M (**) and for raltitrexed at 10⁻⁶ M. (E) Western blots (for three independent samples) evaluating TS expression in G48a, U-251 MG, and SNB-19 GBM cells relative to HL60 cells that are sensitive to F10 at nM concentrations. Overall sensitivity to F10 inversely correlates with TS expression.

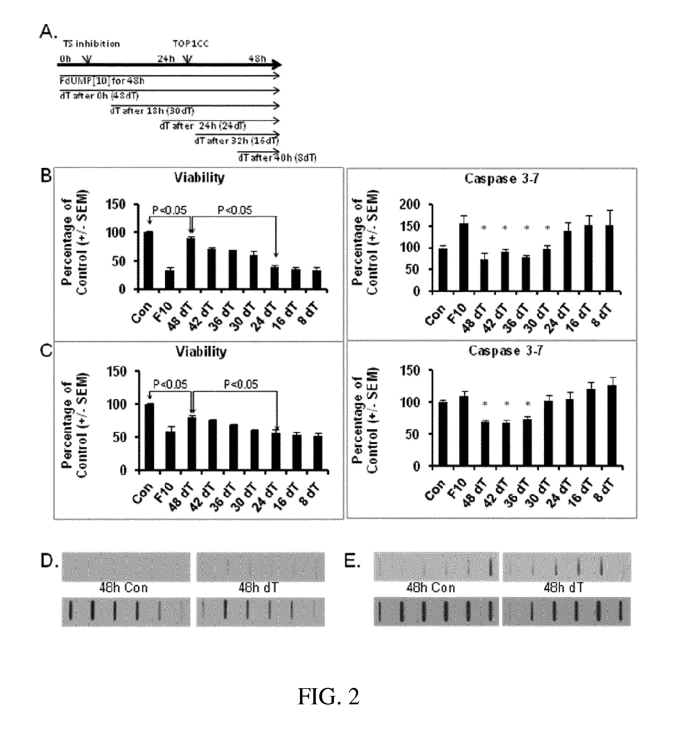

**Figure 2****. F10 Cytotoxic and apoptotic effects are rescued by exogenous Thy only during first replicative cycle.** (A) Timing of Thy-rescue experiments; (B,C) Effect of F10 for 48 h treatment towards (B) SNB19 and (C) U251 cells. Exogenous Thy (dT - 80 µM) was added as a potential rescue agent for the indicated times from end of treatment. For each cell line, the graph on the left represents cell viability while the graph on the right represents apoptosis. 48 h dT rescue in U251 cells was not complete and 24 h rescue was significantly reduced relative to 48 h rescue (p < 0.05). Apoptosis was significantly reduced by Thy rescue for these timepoints indicated with a * (p < 0.05). Thy rescue effects are limited to the first 24 h of F10 treatment. (D,E) In vivo complex of enzyme (ICE) bioassay results demonstrate that F10 induces Top1CC formation in (D) SNB-19 and (E) U-251 MG cells. Exogenous Thy rescues Top1CC if co-administered with F10 for 48 h but is not effective at preventing Top1CC formation if administered during the final 16 h of treatment after cells had committed to or undergone DNA replication and mitosis.

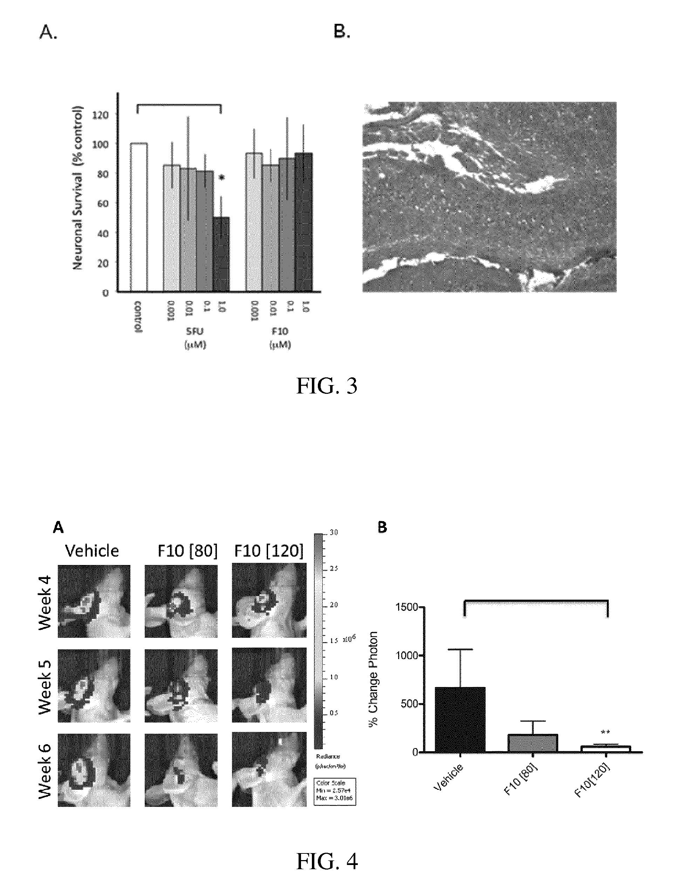

**Figure 3****. F10 is not toxic to primary neuronal cultures and does not damage normal brain tissue upon i.c. administration.** (A) Viability of primary neuronal cells grown in tissue culture following treatment with F10 or 5FU at the indicated doses. 5FU, but not F10, significantly decreased neuronal survival at 1 µM relative to control (p < 0.05). (B) H&E stained section from the brain of mice treated with F10 at 120 mg/kg.
**Figure 4****. F10 treatment results in significant and dose-responsive regression of G48a orthotopic xenografts.** (A) Luciferase signal from F10- and vehicle-treated nude mice at 80 and 120 mg kg doses. Treatment with F10 results in marked decrease in luciferase-signal for treated mice. (B) Mean tumor volumes calculated from the luciferase images. F10 treatment results in regression of G48a xenografts that is highly significant (p < 0.01) relative to vehicle for the 120 mg/kg treatment.

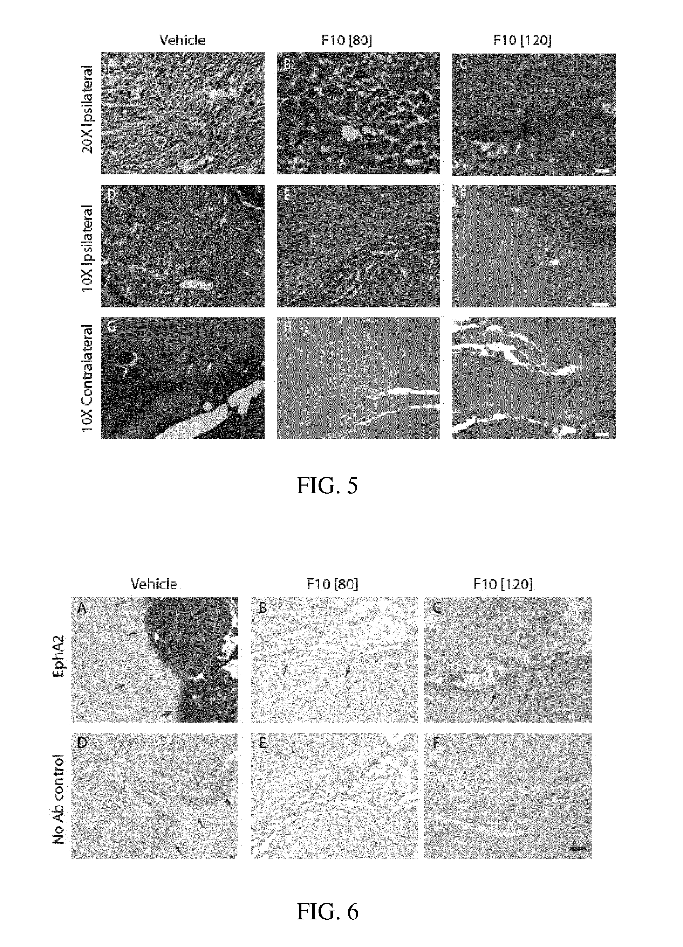

**Figure 5****. F10 treatment results in selective eradication of G48a orthotopic brain tumors in nude mice.** H&E staining from brain sections obtained from nude mice bearing G48a xenografts following treatment with vehicle only (A,D,G) or F10 at 80 (B,E,H) or 120 mg/kg (C,F,I). F10, or vehicle, was administered i.c. over seven days using an osmotic pump. Brain tissue from mice treated with vehicle-only reveals extensive infiltration of GBM cells into non-malignant tissue and a significant tumor mass (arrows point to tumor borders). Treatment with F10 at either 80 or 120 mg/kg resulted in extensive necrosis selectively for malignant cells with no apparent damage for non-malignant cells in the contralateral (contra) side at the level of the hippocampus. Invasive island of cells were observed in contralateral hippocampus of vehicle-treated animals (arrows in G) (Scale bar = 100 µm in C and I, 50 µm in F).
**Figure 6****. F10 Treatment results in selective eradication of EphA2-stained G48a cells.** EphA2 staining from brain sections obtained from nude mice bearing G48a xenografts following treatment with vehicle only (A,D) or F10 at 80 (B,E) or 120 mg/kg (C,F). Strong EphA2 staining is observed for tumor tissue in vehicle-only treated mice with no EphA2 staining in adjacent non-malignant tissue except in isolated invasive cells (arrows indicate tumor borders and point to invasive cells away from tumor core). EphA2 staining is greatly diminished in region of the residual tumor (indicated by arrows) from mice treated with F10 at 80 mg/kg and is absent in mice treated with F10 at 120 mg/kg (Scale bar = 50 µm).

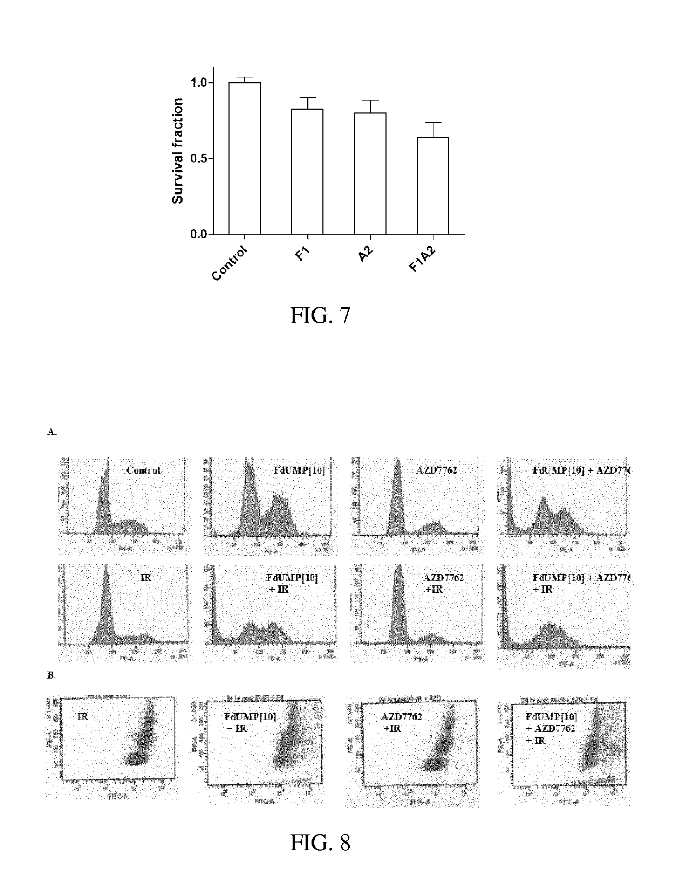

**Figure 7****. Combined treatment with FdUMP[10] and AZD7762 led to a decreased clonogenic survival of U138 cells.** 500-2000 U138 cells were seeded into 60mm dishes and treated with 1nM FdUMP[10] (F1) and/or 10 nM AZD7726 (A2) and the drugs were removed after 72 h. Cells were fixed and stained with violet blue and colonies were counted using a colony counter 10 days post-treatment. FdUMP[10] and AZD7762 treatment resulted in 0.84 and 0.80 survival fractions, respectively. FdUMP[10]/AZD7762 co-treatment resulted in a 0.62 survival fraction exceeding the 0.67 expected based on an additive interaction. The results are consistent with FdUMP[10]AZD7762 being synergistic at decreasing the clonogenicity of U138 cells.
**Figure 8****. Treating U138 cells with FdUMP[10]/AZD7762 + IR results in predominantly apoptotic cell death.** U138 cells were treated with 1nM FdUMP[10] and/or 10 nM of AZD7762 for 9 or 1 h, respectively, prior to irradiation with a single dose of 6 Gy. Cells were harvested 24 h post-irradiation and analyzed for DNA content (A) and cleaved caspase 3 (B) by flow cytometry. (A) DNA histograms showing FdUMP[10] causes G2-arrest. Co-treatment with AZD7762 abrogates G2-arrest and results in cells with sub-G0 DNA content. Irradiation enhances the percentage of cells with sub- G0 content for FdUMP[10] and FdUMP[10]/AZD7762 treatments. (B) Cleaved caspase 3 (x-axis) vs DNA content (y-axis). Vehicle-only (not shown), IR-only, AZD7762-only (not shown) and AZD7762 + IR do not result in significant percent of cleaved caspase 3 while FdUMP[10]+IR and FdUMP[10]/AZD7762+IR treatments result predominantly in apoptotic cells.

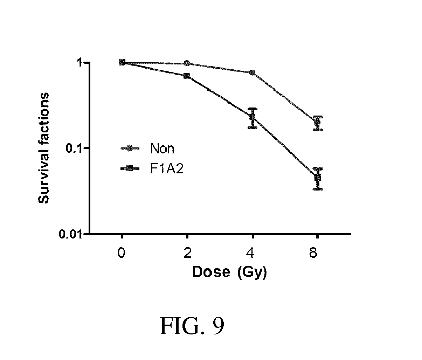

**Figure 9****. Treating human glioma U138 cells with FdUMP[10] and AZD7726 enhanced their radiosensitivity.** 500-2000 U138 cells were seeded into 60mm dishes and treated with 1nM FdUMP[10] (F1) for 9 h and 10 nM AZD7726 (A2) for 1 h prior to irradiation with a single dose of 6 Gy. Cells were fixed and stained with violet blue and colonies were counted using a colony counter at 10 days post-treatment. The study indicates combined treatment U138 with FdUMP[10] and AZD7762 sensitize cells to radiation.

### DETAILED DESCRIPTION OF EMBODIMENTS

The present invention is directed to methods of using an active agent in the treatment of cancers of the central nervous system. The cancer can be a primary or secondary brain cancer. Cancer treatable with embodiments of the present invention include glioma, particularly glioblastoma multiforme. Other brain cancers are also treatable, including, but not limited to, astrocytoma, oligodendroglioma, ependymoma, meningiomas, acoustic neuroma/schwannomas, and medulloblastoma. Also included is neuroblastoma.

In some embodiments, the cancer is a secondary brain cancer which has metastasized from a non-brain cancer.

All references cited are incorporated by reference to the extent they are consistent with the disclosure provided herein.

"Brain cancer" or "brain tumor" may be any stage, grade, histomorphological feature, invasiveness, aggressivity or malignancy of an affected tissue or cell aggregation in any part of the central nervous system (i.e., brain and spinal cord). In some embodiments, the brain tumor is a glioma. In some embodiments, the tumor is an anaplastic astrocytoma, anaplastic oligoastrocytoma or anaplastic oligodendroglioma, in particular, fibrillary astrocytoma WHO grade II, oligoastrocytoma WHO grade II, oligodendroglioma grade II, anaplastic astrocytoma WHO grade III, anaplastic oligoastrocytoma WHO grade III, anaplastic oligodendroglioma grade III or glioblastoma, such as glioblastoma multiforme (*see, e.g.,* US Patent Application Publication No. 2010/0291590).

Gliomas are tumors occurring in the glial cells, which help support and protect critical areas of the brain. Gliomas are the most common type of brain tumor in adults, responsible for about 42% of all adult brain tumors. Gliomas are further characterized by the types of cells they affect, into the categories of astrocytoma (affecting astrocytes), oligodendroglioma (affecting oligodendrocytes), ependymoma (affecting ependymal cells), meningiomas (affecting the meninges), acoustic neuroma/schwannoma (affecting Schwann's cells), and medulloblastoma (affective cells in the cerebellum). *See also* U.S. 2013/0012452 to Basile et al.

Astrocytomas are graded from I to IV depending on the speed of progression. Grade I (pilocytic astrocytoma) is slow growing, with little tendency to infiltrate surrounding brain tissue. Grade II (diffuse astrocytoma) is fairly slow-growing, with some tendency to infiltrate surrounding brain tissue. Grade III (anaplastic/malignant astrocytoma) tumors grow rather quickly and infiltrate surrounding brain tissue. Grade IV (glioblastoma multiforme, GBM) is an extremely aggressive and lethal form of brain cancer. Unfortunately, it is the most common form of brain tumor in adults, accounting for about 67% of all astrocytomas.

Oligodendrogliomas, which make up 4% of brain tumors, mostly affect people over 45 years of age. Some subtypes of this tumor are particularly sensitive to treatment with radiation therapy and chemotherapy. Half of patients with oligodendrogliomas are still alive after five years.

Ependymomas are rare; about 2% of all brain tumors, but are the most common brain tumor in children. They generally do not affect healthy brain tissue and do not spread beyond the ependyma. Although these tumors respond well to surgery, particularly those on the spine, ependymomas cannot always be completely removed. The five-year survival rate for patients over age 45 approaches 70%.

Meningiomas affect the meninges, the tissue that forms the protective outer covering of the brain and spine. One-quarter of all brain and spinal tumors are meningiomas, and up to 85% of them are benign.

Malignant gliomas are a fatal disease with an average life-expectancy following diagnosis of less than one year. The prognosis for patients with high-grade gliomas is very poor, and this is especially so for older patients. Of Americans diagnosed each year with malignant gliomas, about half are alive 1 year after diagnosis, and 25% after two years. Those with anaplastic astrocytoma survive about three years. Glioblastoma multiforme has the worse prognosis, with a life expectancy of less than 9-15 months following diagnosis.

The present invention is primarily concerned with the treatment of human subjects, but the invention may also be carried out on animal subjects, particularly mammalian subjects such as dogs, cats, livestock and horses for veterinary purposes. While subjects may be of any suitable age, the subjects are in some embodiments neonatal, infant, juvenile, adolescent, adult, or geriatric subjects.

"Treat" as used herein refers to any type of treatment that imparts a benefit to a subject, particularly delaying or retarding the progression of the disease or cancer. For example, the treatment may kill or otherwise decrease the number of cells and/or volume of cancerous tissue in the brain or central nervous system, inhibit or slow the progression of the cancer, alleviate side effects such as cognitive abnormalities, etc.

"Pharmaceutically acceptable" as used herein means that the compound or composition is suitable for administration to a subject to achieve the treatments described herein, without unduly deleterious side effects in light of the severity of the disease and necessity of the treatment.

### 1. Active agents.

Active agents used to carry out the present invention are, in general, homo-oligomeric nucleotides of 5-fluorouracil ("poly-FdUMP"), particularly oligonucleotides containing 8, 9, 10, 11, or 12 monomers of 5-fluorodeoxyuridine covalently linked via 3' to 5' phosphodiester linkages. For example, FdUMP[10] (F10) has the structure: 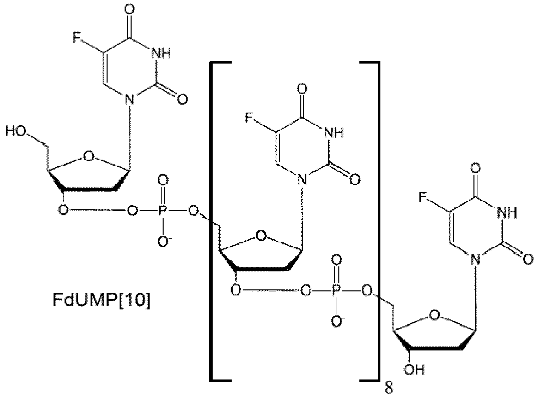 Such compounds are known and described in, for example, U.S. Patents Nos. 5,457,187 and 6,342,485, the disclosures of each of which are incorporated by reference herein in their entirety.

The poly-FdUMP may be provided as the structure presented above, or with modifications, particularly at the 5' and/or 3' end of the polynucleotide, in order to slow their degradation or provide other desirable properties (though not prohibiting the poly-FdUMP to eventually break apart, which is needed for biological activity (*e.g*., as a substrate/inhibitor of thymidylate synthetase, DNA polymerases, and/or DNA repair enzymes such as Top1)).

For example, backbone modifications at the 5' and/or 3' terminal linkages (*e.g.,* first, second, and/or third from the end of the molecule) may be used. Such modifications are known in the art, and include, but are not limited to, modifications in the internuclotide phosphodiester linkage such as methylphosphonate, phosphorothioate, phosphorodithioate, phosphorothiolate, phosphoramidate, etc.

As another example, covalent linkage of the 5' and/or 3' end of the polynucleotide may be used, such as with a levulinyl or acetal levulinyl group, a hydrophobic molecule (*e.g.,* cholesterol), or other molecules that may increase cancer cell uptake (*e.g.,* biotin, folic acid). *See* U.S. Patent No. 5,457,187 to Gmeiner et al. and U.S. Patent No. 6,342,485 to Gmeiner, the disclosures of each of which are incorporated by reference herein.

"Levulinyl" or "Lv" is the group: -C(O)CH₂CH₂C(O)CH₃ (levulinyl, Lv). "Acetal levulinyl" or "ALE" is the group: -CH₂OC(O)CH₂CH₂C(O)CH₃. *See also* U.S. Patent Application Publication No. 2012/0178638 to Damha et al.

Poly-FdUMP has distinct pharmacological and biochemical properties relative to conventional FP drugs (e.g. 5-fluorouracil (5FU) and capecitabine) that may prove advantageous for treating CNS malignancies. *See* Gmeiner et al., Enhanced DNA-directed effects of FdUMP[10] compared to 5FU. Nucleosides Nucleotides Nucleic Acids. 2004;23(1-2):401-410; Liao et al., A novel polypyrimidine antitumor agent FdUMP[10] induces thymineless death with topoisomerase I-DNA complexes. Cancer Res. 2005;65(11):4844-4851; Bijnsdorp et al., Mechanisms of action of FdUMP[10]: metabolite activation and thymidylate synthase inhibition. Oncol Rep. 2007;18(1):287-291.

As a nucleic acid polymer, poly-FdUMP such as FdUMP[10] have a greater size and charge than FP monomers such as 5FU, and likely is retained within the blood brain barrier (BBB) upon intra-cerebral (i.c.) administration. Retention within the BBB is expected to enable high local concentrations of drug that have the potential to result in strong anti-tumor activity, provided a sufficient therapeutic window is present such that the relatively high drug concentrations achieved do not damage non-malignant neuronal cells. *See* Gmeiner, Novel chemical strategies for thymidylate synthase inhibition. Curr Med Chem. 2005;12(2):191-202; Buonerba et al., A comprehensive outlook on intracerebral therapy of malignant gliomas. Crit Rev Oncol Hematol. 2011;80(1):54-68; Debinski et al., Convection-enhanced delivery for the treatment of brain tumors. Expert Rev Neurother 2009;9:1519-1527.

For conventional FP drugs such as 5FU, neurotoxicity largely results from the degradatory metabolite α-fluoro-β-alanine (FBAL). *See* Yamashita at al., Neurotoxic effects of alpha-fluoro-beta-alanine (FBAL) and fluoroacetic acid (FA) on dogs. J Toxicol Sci. 2004;29(2):155-166; Diasio et al., Fluoropyrimidine catabolism. Cancer Treat Res. 1995;78:71-93. As a nanoscale polymer, F10 is expected to be too large to be a substrate for enzymes that degrade 5FU and, as a consequence, F10 is not expected to be neurotoxic by processes that limit the applicability of conventional FPs for treatment of CNS malignancies. As taught herein, F10 is much less cytotoxic than 5FU to normal neuronal cells; however F10 is highly effective for treating GBM *in vivo.*

The active agents disclosed herein can, as noted above, be prepared in the form of their pharmaceutically acceptable salts. Pharmaceutically acceptable salts are salts that retain the desired biological activity of the parent compound and do not impart undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; (b) salts formed from elemental anions such as chlorine, bromine, and iodine, and (c) salts derived from bases, such as ammonium salts, alkali metal salts such as those of sodium and potassium, alkaline earth metal salts such as those of calcium and magnesium, and salts with organic bases such as dicyclohexylamine and N-methyl-D-glucamine.

### 2. Pharmaceutical formulations.

The active agents described herein may be formulated for administration in a pharmaceutical carrier in accordance with known techniques. *See, e.g.,* Remington, The Science And Practice of Pharmacy (9th Ed. 1995). In the manufacture of a pharmaceutical formulation according to the invention, the active agent (including the physiologically acceptable salts thereof) is typically admixed with, *inter alia,* an acceptable carrier. The carrier must, of course, be acceptable in the sense of being compatible with any other ingredients in the formulation and must not be deleterious to the patient. The carrier may be a solid or a liquid, or both, and is preferably formulated with the compound as a unit-dose formulation, for example, a tablet, which may contain from 0.01 or 0.5% to 95% or 99% by weight of the active agent. One or more active agents may be incorporated in the formulations of the invention, which may be prepared by any of the well-known techniques of pharmacy comprising admixing the components, optionally including one or more accessory ingredients.

Further, the present invention provides liposomal formulations of the compounds disclosed herein and salts thereof. The technology for forming liposomal suspensions is well known in the art. When the compound or salt thereof is an aqueous-soluble salt, using conventional liposome technology, the same may be incorporated into lipid vesicles. In such an instance, due to the water solubility of the compound or salt, the compound or salt will be substantially entrained within the hydrophilic center or core of the liposomes. The lipid layer employed may be of any conventional composition and may either contain cholesterol or may be cholesterol-free. When the compound or salt of interest is water-insoluble, again employing conventional liposome formation technology, the salt may be substantially entrained within the hydrophobic lipid bilayer that forms the structure of the liposome. In either instance, the liposomes produced may be reduced in size, as through the use of standard sonication and homogenization techniques.

The liposomal formulations containing the compounds disclosed herein or salts thereof, may be lyophilized to produce a lyophilizate, which may be reconstituted with a pharmaceutically acceptable carrier, such as water, to regenerate a liposomal suspension.

Other pharmaceutical compositions may be prepared, such as aqueous base emulsions. In such an instance, the composition will contain a sufficient amount of pharmaceutically acceptable emulsifying agent to emulsify the desired amount of the compound or salt thereof. Particularly useful emulsifying agents include phosphatidyl cholines, and lecithin.

Further provided are active agents in the form of an implant which provides continuous administration as the implant dissolves and/or the agent is eluted from the implant. The implant may be placed during surgery in accordance with known methods. *See, e.g.,* Perry et al., "Glidel wafers in the treatment of malignant glioma: a systemic review," Curr. Oncol. 14(5): 189-194 (2007).

In addition to active agent(s), the pharmaceutical compositions may contain other additives, such as pH-adjusting additives. In particular, useful pH-adjusting agents include acids, such as hydrochloric acid, bases and/or buffers, such as sodium lactate, sodium acetate, sodium phosphate, sodium citrate, sodium borate, or sodium gluconate. Further, the compositions may contain microbial preservatives. Useful microbial preservatives include methylparaben, propylparaben, and benzyl alcohol. The microbial preservative is typically employed when the formulation is placed in a vial designed for multidose use. As indicated, the pharmaceutical compositions of the present invention may be lyophilized using techniques well known in the art.

### 3. Dosage and routes of administration.

As noted above, the present invention provides pharmaceutical formulations comprising active agents (including the pharmaceutically acceptable salts thereof), which may be provided in pharmaceutically acceptable carriers for administration. The carrier in some embodiments is a liquid carrier suitable for infusion. The carrier may be, for example, an aqueous carrier (e.g., comprising water, such as a saline solution).

In some embodiments, the active agent is administered directly into the brain (*i.e.,* within the blood brain barrier) and/or other portions of the central nervous system of a subject. In some embodiments, the active agent is administered to the subject intra-cerebrally. In some embodiments, the active agent is administered to the subject by intracerebroventricular infusion. In some embodiments, the active agent is administered by intrathecal delivery. In some embodiments, the active agent is administered by convection-enhanced delivery.

Convection-enhanced delivery (CED) is the continuous injection under positive pressure of a fluid containing a therapeutic agent. In the central nervous system (CNS), this delivery technique circumvents the blood-brain barrier in delivering agents. *See, e.g.,* US 2012/0041394 to Haider et al.; US 2012/0209110 to Bankiewicz et al. CED uses a fluid pressure gradient established at the tip of an infusion catheter and bulk flow to propagate substances within the extracellular fluid space. CED allows the extracellularly-infused material to further propagate via the perivascular spaces and the rhythmic contractions of blood vessels acting as an efficient motive force for the infusate. As a result, a higher concentration of drug can be distributed more evenly over a larger area of targeted tissue than would be seen with a simple injection. CED has been clinically tested in the fields of neurodegenerative diseases and neurooncology, and is useful in a broad field of applications, such as the delivery of small molecules, macromolecules, viral particles, magnetic nanoparticles, and liposomes.

In some embodiments, the active agent is administered in combination with radiation therapy. In some embodiments, the active agent is administered in combination with surgery to remove all or part of the cancerous tissue. In some embodiments, the active agent is administered in combination with another, different chemotherapy agent.

Other agents useful for chemotherapy according to the present invention include, but are not limited to, another thymidylate synthase inhibitor, an EGFR inhibitor (*e.g*., an EGFR ligand, an EGFR-targeting antibody, 3-(4-Isopropylbenzylidenyl)indolin-2-one, Afatinib, AG-490, AG 1478, AG 555, AG 825, Canertinib, CL-387,785, Dacomitinib, Erlotinib, etc.), a casein kinase (ck) 1/2 inhibitor (*e.g*., AZD7762, D4476), temozolomide (TMZ), or any combination thereof. *See, e.g.,* U.S. Patent Application Publication No. 2012/0316155 to Baldino et al.; U.S. Patent No. 8,334,293, which are incorporated by reference herein.

Apart from the poly-FdUMP agent, other agents are known to inhibit thymidylate synthase (TS) and may be used in combination therewith, particularly for cancers that express high levels of TS, such as the brain cancers described herein. As opposed to other cancers for which TS activity is not particularly elevated, cancers expressing high TS activity, such as GBM, may particularly benefit from this combination. For example, nucleic acid-based/RNA-targeting agents such as RNAi, siRNA, shRNA, etc., may be used. See, e.g., U.S. 2012/0016012 to Wada et al.; U.S. 2011/0003879 to Vincent et al.; U.S. 2008/0145313 to Watson et al.; U.S. 2012/0301537 to Ishida et al.; Wu et al., "Development of modified siRNA molecules incorporating 5-fluoro-2'-deoxyuridine residues to enhance cytotoxicity," Nucleic Acids Res. (2013), which are incorporated by reference herein. In some embodiments, the thymidylate synthase inhibitor may be coupled to the poly-FdUMP. In some embodiments, the thymidylate synthase inhibitor may be covalently coupled to the poly-FdUMP.

An EGFR inhibitor includes, but is not limited to, an EGFR ligand, for example, EGF or a mimetic thereof, an antibody or fragment thereof, etc. *See, e.g.,* U.S. 2003/0211112 to Debinski; U.S. 2013/0041136 to Beckmann et al., which are incorporated by reference herein. In some embodiments, the EGFR inhibitor may be coupled to the poly-FdUMP to promote delivery thereof to cells expressing an elevated level of EGFR, such as the brain cancer cells described herein. In some embodiments, the EGFR inhibitor may be covalently coupled to the poly-FdUMP.

As used herein, the administration of two or more therapies (inclusive of active agents, other chemotherapeutics, radiation therapy, etc., or any combination thereof) "in combination" means that the two are administered closely enough in time that the administration of or presence of one alters the biological effects of the other. The therapies may be administered simultaneously (concurrently) or sequentially.

Simultaneous administration of the agents may be carried out by mixing the agents prior to administration, or by administering the agents at the same point in time but at different anatomic sites or using different routes of administration, or administered at times sufficiently close that the results observed are indistinguishable from those achieved when the agents are administered at the same point in time. Simultaneous administration of one or more agents with radiation may be carried out by administering the compounds at the same point in time as the radiation is applied, or at times sufficiently close that the results observed are indistinguishable from those achieved when the compounds and radiation are administered at the same point in time.

Sequential administration of the agents may be carried out by administering the agents at different points in time, *e.g.,* an active agent at some point in time prior to or after administration of one or more other chemotherapeutics, such that the administration of agent enhances the therapeutic effect of cancer treatment. In some embodiments, an active agent is administered at some point in time prior to the initial administration of another chemotherapeutic or other therapy. Alternatively, the other therapeutic or therapy may be administered at some point in time prior to the administration of an active agent, and optionally, administered again at some point in time after the administration of an active agent.

In some embodiments, the active agent is administered to the subject in an amount of from 0.1, 0.5, 1 or 5, to 10, 25, 50 or 100 mg/kg. In some embodiments, the active agent is administered to the subject in an amount of from 50, 100, or 200 to 300 or 400 mg/kg. In some embodiments, the active agent may be administered 1 to 5, 6, or 7 times weekly (*e.g.*, for a period of from 4 to 6 weeks per cycle). In some embodiments, the active agent is administered as a continuous or substantially continuous infusion for at least 2 or at least 3 days. In some embodiments, the active agent is administered as a continuous or substantially continuous infusion in a range of from 5, 6, 7 or 8, to 10, 12, 14 or 16 days. For example, the active agent may be administered as a continuous infusion for 7-14 days, which may be repeated as desired (*e.g*., repeated every 4-6 weeks).

Because poly-FdUMP does not cross the blood brain barrier, the agent may be advantageously kept at the intended site of action upon infusion into the central nervous system tissues.

The present invention is explained in greater detail in the following non-limiting Examples.

### EXAMPLE 1: Selective Reduction of G48a Orthotopic Xenografts with the poly-FdUMP F10

The anti-tumor activity of F10 towards an orthotopic model of GBM was evaluated to assess F10 as a treatment for GBM patients.

**Methods.** The effects of F10 on thymidylate synthase (TS) inhibition and DNA Topoisomerase 1 (Top1) cleavage complex formation were evaluated using TS activity assays and *in vivo* complex of enzyme (ICE) bioassays. Cytotoxicity of F10 towards normal brain was evaluated using cortices from embryonic (day 18) mice. F10 was delivered by intra-cerebral (i.c.) administration and prospective anti-tumor response towards luciferase-expressing G48a GBM cells was evaluated using IVIS imaging. Histological examination of tumor and normal brain tissue was used to assess anti-tumor activity.

**Results.** F10 is cytotoxic towards G48a, SNB-19, and U-251 MG GBM cells through dual targeting of TS and Top1. F10 is not toxic to primary neuronal astrocyte cultures, and F10 was well-tolerated upon intra-cerebral administration resulting in significant regression of G48a tumors that was dose-responsive. Histological analysis from F10-treated mice revealed tumors were essentially completely eradicated in F10-treated mice while vehicle-treated mice displayed substantial infiltration into normal tissue.

**Conclusions.** F10 displays strong efficacy for GBM treatment with minimal toxicity upon i.c. administration, establishing F10 as a promising agent for treating GBM in human patients.

The mechanism of cell death in AML cells following F10 treatment involves dual targeting of TS and DNA Topoisomerase 1 (Top1) with Top1 cleavage complexes (Top1CC) being the principal cytotoxic lesions. (Pardee et al., Unique dual targeting of thymidylate synthase and topoisomerase 1 by FdUMP[10] results in high efficacy against AML and low toxicity. Blood. 2012; 119(15):3561-3570; Gmeiner et al., Enhanced DNA-directed effects of FdUMP[10] compared to 5FU. Nucleosides Nucleotides Nucleic Acids. 2004;23(1-2):401-410; Liao et al., A novel polypyrimidine antitumor agent FdUMP[10] induces thymineless death with topoisomerase I-DNA complexes. Cancer Res. 2005;65(11):4844-4851; Gmeiner, Novel chemical strategies for thymidylate synthase inhibition. Curr Med Chem. 2005;12(2):191-202; Jennings-Gee et al., Replication-Dependent Irreversible Topoisomerase 1 Poisoning is Responsible for FdUMP[10] Anti-Leukemic Activity. Exp Hem. 2013. PMID:23085462 In Press.)

Top1-poisoning by F10 requires the misincorporation of FdUTP into genomic DNA, and thus F10-induced Top1CC formation occurs selectively for proliferating cells. FdUTP incorporation into genomic DNA is enhanced under thymineless conditions resulting from FdUMP-induced thymidylate synthase (TS) inhibition. Thymineless conditions may also inhibit repair of Top1-induced DNA damage. F10 is chemically distinct from conventional Top1 poisons, such as camptothecins (CPTs) that are neurotoxic by a Top1-independent mechanism, and may also cause neurotoxicity at supraphysiological concentrations via on-target effects. (Uday Bhanu and Kondapi, Neurotoxic activity of a topoisomerase-I inhibitor, camptothecin, in cultured cerebellar granule neurons. Neurotoxicology 2010;31(6):730-737; Morris and Geller, Induction of neuronal apoptosis by camptothecin, an inhibitor of DNA topoisomerase-I: evidence for cell cycle-independent toxicity. J Cell Biol. 1996;134(3):757-770.) While not wishing to be bound to a particular theory, the requirement for cell proliferation to induce Top1CC in F10-treated cells is expected to prevent damage to non-proliferating cells and to provide a selective therapeutic advantage for the treatment of aggressive malignancies, such as GBM, as is demonstrated in the present studies.

The present studies probed to what extent the high degree of sensitivity of GBM cell lines to F10 may achieve strong anti-tumor activity in a realistic animal model of GBM. An orthotopic xenograft model of GBM was used in which *luc*-transfected G48a GBM cells were injected directly into the brains of immunocompromised mice. The G48a cell line was established from cells isolated from a patient with multi-foci GBM. (Debinski and Gibo, Fos-related antigen 1 modulates malignant features of glioma cells. Mol Cancer Res. 2005;3(4):237-249; Wykosky et al., A novel, potent, and specific ephrinA1-based cytotoxin against EphA2 receptor expressing tumor cells. Mol Cancer Ther. 2007;6(12 pt.1):3208-3218.) I.C. injection of G48a cells results in formation of a highly invasive and highly proliferative malignancy that replicates the characteristic features of the human disease.

The i.c. administration of F10 results in dramatic regression of G48a tumors, indicating that F10 is an efficacious *in vivo* treatment.

### Materials and Methods

***Cell Culture and ICE Bioassay.*** G48a and primary explant cells derived from patient GBM tumors were grown in RPMI 1640 (Lonza, Rockland, ME) supplemented with 10% FBS and glucose (2 g/L). G48a-luciferase cells were produced by lentiviral infection using a GFP-tagged vector. Cells were selected using blastocydin (10 µg/mL). U-251 MG and SNB-19 cells were obtained from ATCC and grown in RPMI 1640 (Lonza, Rockland, ME) supplemented with 10% FBS and glucose (2 g/L). U-251 MG cells were grown in DMEM supplemented with 10% FBS and non-essential amino acids. *In vivo* complex of enzyme (ICE) bioassays were completed using methods similar to those previously described. (Liao et al., A novel polypyrimidine antitumor agent FdUMP[10] induces thymineless death with topoisomerase I-DNA complexes. Cancer Res. 2005;65(11):4844-4851.) GBM cells were incubated with F10 at the indicated concentrations for 0-48 hrs. Thymidine (Thy) rescue was accomplished by adding Thy at 80µM, as the 20 µM dose previously described (Wang et al., Mechanisms of acquired chemoresistance to 5-fluorouracil and tomudex: thymidylate synthase dependent and independent networks. Cancer Chemother Pharmacol. 2007;59(6):839-845) was not effective for rescue. Cell samples were counted and equalized for cell number. Primary antibody (mouse anti-human DNA Top I, BD Pharmingen) was added at 1:500 dilution. Secondary antibody (Cell Signaling) was used at 1:1000. ECL Lightning Plus (PerkinElmer) was used for detection of the Top1CC.

***TS Catalytic Activity Assays**.* TS assays were conducted using procedures similar to those previously described. (Gmeiner et al., Enhanced DNA-directed effects of FdUMP[10] compared to 5FU. Nucleosides Nucleotides Nucleic Acids. 2004;23(1-2):401-410.) GBM cells were plated at a density of 1.5 x10⁶ cells in 100 mm² plates. Cells were grown overnight in RPMI 1640 medium with 10% FBS. Either 5FU (Sigma), F10, or raltitrexed were then added at the indicated concentrations and the cells were incubated for 8, 16, 24, or 48 hrs, harvested, and lysed by freeze-fracturing. Following centrifugation of cell lysates, supernatants were assayed for protein content and TS catalytic activity as previously described. (Gmeiner et al., Enhanced DNA-directed effects of FdUMP[10] compared to 5FU. Nucleosides Nucleotides Nucleic Acids. 2004;23(1-2):401-410.)

***Caspase Activity and Viability Assays.*** Caspase 3/7, 8, and 9 activity and cell viability assays were performed using Promega Caspase-Glo® Assay reagents for caspase activity and CellTiter-Glo® Luminescent Cell Viability Assay reagents for cell viability. Experiments were performed per manufacturer's instructions. Briefly, 2x10⁵ cells were plated in 24 well plates in triplicate, and drug treatments started 20-24 hrs after plating. Cells were re-suspended before 50-100µL aliquots were taken at the indicated times and mixed with an equal volume of assay kit reagent in a 96-well white plate. The plates were then incubated at RT for 30-60 min per instructions before being read using a Tecan GENios luminescence plate reader. Apoptosis data was normalized for cell number using viability data. (Sharpe et al., FGFR signaling promotes the growth of triple-negative and basal-like breast cancer cell lines both in vitro and in vivo. Clin Cancer Res. 2011;17(16):5275-5286.)

***Intracranial GBM model.*** G48a-luc were suspended into Hanks Balanced Salt Solution to a density of 10⁵ cells/µL. Nude (*nu*/*nu*) 7-week old mice were obtained from NCI. Mice were anesthetized using a ketamine/xylazine mixture and placed on a small-animal, head-holding frame. A scalp incision was made to determine the drill-hole location, 1 mm right and 1 mm anterior to lambda. A 27-gauge needle attached to a 10 mL sterile Hamilton syringe (Hamilton, Reno, NV) was stereotactically inserted 3 mm below the dural surface and cells were injected into the deep white matter of the posterior thalamus. Cells were injected in a total of 5 µL over a five minute period. The syringe was removed two minutes after the injection was completed, retracting it at a rate of 0.5 mm per minute. After removal of the syringe, the hole was covered with cranio-plastic liquid. Animals were sutured, allowed to recover on a heating pad, and returned to their cages. Animal weight and behavior was monitored every three days.

***Animal groups and treatments**.* Starting on day 21 after tumor cell implantation, mice were treated by intra-cerebral (i.c.) infusion (0.5 µL/h) of F10 at 3 concentrations (80, 120 and 160 mg/kg) for a duration of 7 days. Drug was prepared in phosphate buffered saline. Osmotic pumps (ALZET® model 1007D, Alzet, Cupertino, CA) were primed in sterile saline overnight at 37 °C according to manufacturer's specification. The pumps were then coupled to brain infusion kits (ALZET® model 3). Animals were anesthetized using a ketamine/xylazine mixture and placed on a small-animal head holding frame, and the location of initial injection (1 mm right and 1 mm anterior to lambda) was retraced. The cannula was then inserted to a depth of 3 mm and secured with cranio-plastic adhesive and the pump was placed subcutaneously between the shoulder blades. Animals were sutured, allowed to recover and evaluated for any motor deficits resulting from the surgical procedure. Each treatment group had 5 animals. Animals were euthanized 24 hours post-treatment when body weight loss was above 12% or when behavior prevented normal activity.

***IVIS imaging.*** Tumor growth was monitored by evaluating bioluminescence (Hochgrafe and Mndelkow, Making the brain glow: in Vivo bioluminescence imaging to study neurodegeneration. Mol Neurobiol. 2012;PMID 23192390) using the IVIS Lumina II imaging system (Xenogen Corporation, Alameda, CA). Animals received an intraperitoneal (i.p.) injection of D-luciferin (150 mg/kg, stock solution 15 mg/mL in sterile PBS, Goldbio, St. Louis, MO). After 10 min, animals were anesthetized with isofluorane until non-responsive, and then placed in the imaging chamber. Three bioluminescent imaging acquisitions were collected at different exposure times (10, 60, 300 sec). Mice were allowed to recover and returned to their cages. Data were analyzed based on total photon flux emission (photons) in the region of interest over the intracranial space using Living Image software (Xenogen Corp.).

***Tissue Processing and Immunohistochemistry**.* Animals were deeply anesthetized with an i.p injection of ketamine/xylazine and fixed by transcardial perfusion through the heart with PBS (pH = 7.4) followed by 4% paraformaldehyde in PBS (PFA). Brains were removed and fixed overnight in 4% PFA. Brains were then cryoprotected in 30% sucrose-PBS at 4°C for 2-3 days. Brains were embedded in tissue-freezing medium (Triangle Biomedical Sciences, Durham, NC) and sections were cut to a thickness of 10 µm, thawed onto SuperFrost Plus slides (Fisher, Pittsburgh, PA) and kept at -20 °C until further processing. Sections from vehicle- and F10-treated animals were thawed prior to exposure with primary antibodies. Endogenous peroxidase activity and non-specific biotin was quenched with Peroxide Blocking Kit and Biotin Blocking Kit, respectively (ScyTek Laboratories, Logan, UT). Antigen retrieval was performed with 10 mM sodium citrate buffer, pH 6.0, by microwaving for 5 min. Slides were blocked with SuperBlock (ScyTek) and incubated with polyclonal EphA2 antibody (R&D Systems, Minneapolis, MN) overnight at 4°C. Slides were washed with PBS followed by incubation with biotinylated anti-goat antibody for 15 min, then Avidin-HRP for 20 min (ScyTek). Visualization with NovaRED™ (Vector® Labs) was performed and allowed to develop for 2-10 min. Slides were counterstained with hematoxylin for 1 min, dehydrated, and mounted with Permount™ (Fisher). Additionally, sections were processed for hematoxylin and eosin staining using standard procedures.

***Cortical Neuronal Cultures***: Cortical neuronal cultures were prepared using previously described protocols. (Hockfield et al., Primary Cultures from the Central Nervous System. In: Molecular Probes of the Nervous System. Plainview, NY: Cold Spring Harbor Laboratory Press, 1993: 34-55.) In brief, cortices were isolated from embryonic day 18 mice. The tissue was further dissected into smaller pieces. After washes in cold PBS without Ca⁺²/Mg⁺², tissue was incubated in 0.05% trypsin in PBS without Ca⁺²/Mg⁺². Tissue was then dissociated in NB media using a blue-tip pipette. Dissociated cells were layered onto a 4% BSA cushion to remove debris. Cells were plated in NB media at a density 4 X 10⁴ cells/well in a 24 well dish. After 24 hours, media was removed and replaced with fresh media with or without 5FU or F10. After 72 hours in culture, surviving neurons were counted. To be counted a cell had to have a phase-bright cell body and at least one neurite that was at least two times the diameter of the soma. Results are expressed as percent control (Mean ± SD) where control represents cultures without the addition of 5FU or F10 (n= 4 individual experiments with at least two wells per condition). Statistical significance was determined with a one-way ANOVA followed by the Bonferroni post-hoc test.

### Results

**F10 is Cytotoxic Towards GBM Cells.** Analysis of data from the NCI 60 cell line screen indicated that F10 (NSC-697912) had activity against several of the CNS malignancies included in the screen with the GI₅₀ values for three GBM cell lines (SF-268, SF-295, and SF-539) being at or below the lowest concentration of F10 evaluated in these assays (∼5 nM). (Gmeiner et al., Genome-wide mRNA and microRNA profiling of the NCI 60 cell-line screen and comparison of FdUMP[10] with fluorouracil, floxuridine, and topoisomerase 1 poisons. Mol Cancer Ther. 2010;9(12):3105-3114.) Other CNS malignancies included in the NCI 60 cell line screen, including the SNB-19 and U-251 MG evaluated in these studies, were also sensitive to F10 at sub-micromolar concentrations (GI₅₀ = 1.70 x 10⁻⁷; 1.15 x 10⁻⁷). The cytotoxic mechanism of F10 towards G48a, SNB-19, and U-251 MG cells was evaluated to gain insight into the processes by which this agent may be effective for GBM treatment and to determine to what extent GBM cells respond differently to F10 relative to other types of malignant cells investigated previously. Although not exceptionally sensitive to F10, the G48a cell line (IC₅₀ ∼ 1 x 10⁻⁶ M; **Fig. 1C**) was selected for further study *in vivo* because G48a cells form a highly invasive and rapidly growing tumor upon injection into the brains of immunocompromised mice. (Debinski and Gibo, Fos-related antigen 1 modulates malignant features of glioma cells. Mol Cancer Res. 2005;3(4):237-249.) This orthotopic GBM model replicates several of the challenging features associated with treatment of the human disease in that it is highly infiltrative and rapidly growing.

**F10 Targets TS and Top1 in GBM Cells.** Recent studies from our laboratory demonstrated strong *in vivo* efficacy for F10 in AML treatment with the cytotoxic mechanism towards AML cells involving dual targeting of TS and Top1. (Pardee et al., Unique dual targeting of thymidylate synthase and topoisomerase 1 by FdUMP[10] results in high efficacy against AML and low toxicity. Blood. 2012; 119(15):3561-3570.) TS activity and the ability of exogenous thymidine (Thy) to rescue the cytotoxic effects of F10 were evaluated to determine to what extent F10 induced thymineless death towards GBM cells **(****Fig. 1****).** F10 inhibited TS completely in G48a, U-251 MG, and SNB-19 GBM cells **(****Fig. 1D****),** although higher F10 concentrations were required for G48a and U-251 MG cells than in our previous studies with AML and prostate cancer cells. (Pardee et al., Unique dual targeting of thymidylate synthase and topoisomerase 1 by FdUMP[10] results in high efficacy against AML and low toxicity. Blood. 2012; 119(15):3561-3570; Gmeiner et al., Enhanced DNA-directed effects of FdUMP[10] compared to 5FU. Nucleosides Nucleotides Nucleic Acids. 2004;23(1-2):401-410.) The folate analog Raltitrexed (ZD 1694) (Graham-Cole et al., An evaluation of thymidine phosphorylase as a means of preventing thymidine rescue from the thymidylate synthase inhibitor raltitrexed. Cancer Chemother Pharmacol. 2007;59(2):197-206) also effectively inhibited TS in these GBM cells **(****Fig. 1D****).** Western blots revealed a strong inverse correlation between TS expression and F10 sensitivity **(****Fig. 1E****).**

The contribution of TS inhibition for F10-induced cytotoxicity was then evaluated using thymidine(Thy)-rescue. (Wang et al., Mechanisms of acquired chemoresistance to 5-fluorouracil and tomudex: thymidylate synthase dependent and independent networks. Cancer Chemother Pharmacol. 2007;59(6):839-845.) Recent studies from our laboratory demonstrated exogenous Thy rescued AML cells from F10 if Thy was administered before DNA replication occurred, but that Thy could not rescue cells that had entered a second replicative cycle. (Jennings-Gee et al., Replication-Dependent Irreversible Topoisomerase 1 Poisoning is Responsible for FdUMP[10] Anti-Leukemic Activity. Exp Hem. 2013. PMID:23085462 In Press.) These results are consistent with Thy competing with 5-fluoro-2'-deoxyuridine (FdU) for incorporation into DNA but Thy not being able to replace FdU (via a repair process) in newly synthesized DNA. TS inhibition depletes intracellular Thy resulting in enhanced FdU incorporation into DNA. Similar effects were observed in GBM cells as were observed previously in AML cells. Thy was effective at reversing the cytotoxic and apoptotic effects of F10 during the first 18 h of treatment **(****Fig. 2A****)** but was not effective at later time points after most cells had committed to or undergone DNA replication **(****Fig. 2B,C****).** Previous studies have established that Top1 cleavage complexes (Top1CC) occur in cells that have incorporated FdU into genomic DNA and that Top1CC are subsequently converted into DNA double-strand breaks, and these are the lethal lesions arising from F10 treatment. Top1CC were also detected in GBM cells following F10 treatment **(****Fig. 2D,E****)** and exogenous Thy was able to reverse Top1CC formation only if provided prior to DNA replication. The results are consistent with F10 cytotoxicity arising from the dual targeting of Top1 and TS in GBM cells.

**F10 Displays Minimal Toxicity Towards Primary Cortical Neurons.** Strong differential cytotoxicity towards malignant cells relative to non-malignant cells is critical for drugs or drug-candidates to be useful for cancer treatment. To gain insight into the sensitivity of normal brain tissue to F10, the cytotoxicity of F10 towards primary cortical neuronal cultures from mice was evaluated. The conventional FP drug 5FU was also evaluated in this study as this FP has been shown in previous studies to be toxic to normal neuronal cells. (Han et al., Systemic 5-fluorouracil treatment causes a syndrome of delayed myelin destruction in the central nervous system. J Biol. 2008;7(4):12.) Establishing a toxicity and efficacy advantage for F10 relative to 5FU would demonstrate the benefits of F10 relative to conventional FP therapeutics for treating CNS malignancies. The results are shown in **Fig. 3****.** F10 treatment at concentrations as high as 1 µM resulted in no significant reduction in viability for primary neurons. In contrast, 5FU treatment at 1 µM resulted in approximately 50% decreased viability for cortical neurons relative to control. The same doses were also tested on primary astrocyte cultures with no apparent differences in viability between treated and control cultures (data not shown). It is important to note that the 1 µM dose of F10 consists of 10-times the FP content relative to the 1 µM dose of 5FU due to the stochiometry of F10. The 1 µM dose also exceeded the GI₅₀ value for all CNS malignancies included in the NCI 60 cell line panel and is similar to the IC₅₀ for F10 towards G48a cells. Thus, F10 displays a substantial therapeutic window with preferential cytotoxicity towards malignant cells. These toxicity studies also accentuate the mechanistic distinction between F10 and conventional FPs.

**F10 is Well-Tolerated Upon i.c. Administration.** The nanoscale size of F10 (length ∼ 3 nm), as well as its multiple negative charges, make penetration of the BBB following systemic delivery an inefficient process. Conversely, intra-cranial (i.c.) administration of F10 is expected to result in high local concentrations that may be therapeutically beneficial, particularly in light of the relatively strong cytotoxicity towards GBM cells and the minimal toxicity of F10 towards primary cortical neurons **(****Fig. 3A****).** Dose-finding studies in nude mice were performed to identify doses that were sufficiently well-tolerated to be evaluated in efficacy studies. F10 was dissolved in PBS and administered i.c. using an Alzet osmotic mini-pump over a 7 day period. Vehicle-only served as a control. It was found that F10 at doses up to 200 mg/kg administered over 7 days were well-tolerated and did not cause damage to normal brain tissue **(****Fig. 3B****).** These dose levels are considerably lower than those used in recent leukemia studies in which F10 was administered systemically (200 mg/kg qodx4) (Pardee et al., Unique dual targeting of thymidylate synthase and topoisomerase 1 by FdUMP[10] results in high efficacy against AML and low toxicity. Blood. 2012; 119(15):3561-3570), consistent with F10 being retained within the BBB upon i.c. administration. At a dose of 200mg/kg over 7 days mice appeared light-sensitive, with eyes partially closed, and were more lethargic than mice treated with vehicle-only, although morbidity was not deemed serious enough to warrant removal from study.

**F10 Treatment Results in Significant Regression of GBM Xenografts.** GBM is a challenging malignancy to treat, in part because it is highly infiltrative and rapidly growing. Effective chemotherapeutic treatment requires strong differential cytotoxicity with preferential killing of malignant cells. F10 displayed no toxicity towards primary neuronal cultures at concentrations that were cytotoxic towards GBM cells in tissue culture. G48a orthotopic xenografts were formed in nude mice and the anti-tumor activity for F10 administered at 80, 120, and 160 mg/kg over 7 days was evaluated by IVIS imaging **(****Fig. 4A****).** The results for the 80 and 120 mg/kg treatment are summarized in **Fig. 4****B.** Tumors grew rapidly in mice receiving vehicle-only with mean tumor volumes increasing approximately 600% over the course of the study. In contrast, i.c. administration of F10 resulted in significant tumor regression **(****Fig. 4B****).** Mean tumor volumes (measured by %-change in photon emission) for mice treated with 120 mg/kg F10 were significantly decreased relative to control (p < 0.01). All mice receiving F10 treatment displayed tumor regression. Interestingly, mice treated with 160 mg/kg F10 did not display decreased luminescent signal as was observed for the other treatment groups although histology studies demonstrated a significant treatment effect for this tumor group. Luminescence was observed originating from the ALZET® pump for the 160 mg/kg treatment group suggesting higher concentrations of F10 either are luminescent or simulate luminescence in the context of IVIS imaging (data not shown).

**F10 is Selectively Cytotoxic Towards GBM Cells *in vivo.*** Histological examination of brain tissue extracted from mice treated either with F10 or vehicle control revealed that F10 treatment caused selective death of GBM cells with no apparent damage to normal brain tissue. G48a cells produced highly infiltrative and rapidly growing tumors in the brains of immunocompromised mice with tumors localized to the hemisphere where cells were injected **(****Fig. 5A,D****).** H&E staining of brain sections from vehicle- and F10-treated mice demonstrated that F10 treatment resulted in extensive areas of necrosis selectively within tumor tissue and only in the tumor-bearing side of the brain **(****Fig. 5A-F****).** The extent of F10-induced necrosis was dose-dependent with the 120 mg/kg dose **(****Fig. 5C,F****)** inducing greater tumor cell necrosis than the 80 mg/kg dose **(****Fig. 5B,E****).** There was no observable necrosis in the contralateral side of brains from F10-treated mice **(****Fig. 5G**-I).

To further validate selective death of malignant cells *in vivo,* EphA2-staining of brain sections was performed **(****Fig. 6****).** EphA2 is a brain tumor-specific antigen that is useful for tumor targeting as well as tumor imaging. (Wykosky and Debinski, The EphA2 receptor and ephrinA1 ligand in solid tumors: function and therapeutic targeting. Mol Cancer Res. 2008;6(12):1795-1806.) EphA2 staining of tissues from vehicle-only treated mice revealed extensive regions of tumor mass in the tumor-bearing side of the brain **(****Fig. 6A****),** while the contralateral side did not display any EphA2-positive cells (data not shown). In contrast, sections from the tumor-bearing side of mice treated with F10 at 120 mg/kg showed no EphA2 staining, even in the necrotic tissue that was the remnants of the malignant mass, while only trace levels of EphA2 positive staining were detected in the ventricle of mice treated with F10 at 80 mg/kg **(****Fig. 5B,C****).** As with vehicle-only treated mice, no EphA2 staining was detected in the contralateral sides of brains from F10-treated mice.

### Discussion

Analysis of data from the NCI 60 cell line screen indicated cells derived from human CNS malignancies were particularly sensitive to F10 treatment with nanomolar potency towards several cell lines (e.g. SF268) and a remarkably large differential sensitivity for F10 relative to 5FU (∼10,000-fold for several GBM cell lines). In this work, the anti-tumor activity of F10 towards a G48a orthotopic xenograft model of GBM was evaluated. GBM is particularly challenging for treatment, in part, because GBM cells are highly invasive and rapidly proliferating. G48a cells were selected for *in vivo* studies because these cells form orthotopic tumors in nude mice and the resulting tumors display the aggressive, infiltrating characteristics of the human disease. The results demonstrate that F10 is highly effective not only at reducing the growth rate of G48a xenografts *in vivo,* but F10 also actually induces significant tumor reduction **(****Fig. 4****).** Tumor reduction was achieved in a dose-dependent manner based on luciferase activity with histological analysis indicating extensive necrosis **(****Fig. 5****)** and essentially complete tumor eradication based on elimination of EphA2 staining **(****Fig. 6****).** Importantly, this dramatic anti-tumor effect was achieved with no apparent damage to normal brain tissue **(****Fig. 5****).**

One of the more intriguing findings is that the strong anti-tumor activity for F10 occurs with no apparent damage to normal brain tissue, including brain tissue proximal to the malignancy. These *in vivo* results are consistent with the lack of toxicity for F10 towards primary neuronal cultures **(****Fig. 3****)** and are in stark contrast to the cytotoxicity of F10 towards GBM cells **(****Fig. 1****).** Previous studies have shown that F10 selectively targets replicating cells with the lethal lesions being DNA DSBs generated from Top1 cleavage complexes trapped in front of advancing replication forks. (Liao et al., A novel polypyrimidine antitumor agent FdUMP[10] induces thymineless death with topoisomerase I-DNA complexes. Cancer Res. 2005;65(11):4844-4851.) The lack of toxicity for F10 towards these cells is consistent with mature neuronal cells having low proliferative capacity. In contrast, the conventional FP 5FU is considerably more cytotoxic towards primary neuronal cultures than F10 **(****Fig. 3****),** likely as a consequence of RNA-mediated effects or degradatory metabolites such as FBAL **(****Fig. 1B****)** that are highly neurotoxic towards dogs. (Pritchard et al., Inhibition by uridine but not thymidine of p53-dependent intestinal apoptosis initiated by 5-fluorouracil: evidence for the involvement of RNA perturbation. Proc Natl Acad Sci USA. 1997;94(5):1795-1799; Yamashita et al., Neurotoxic effects of alpha-fluoro-beta-alanine (FBAL) and fluoroacetic acid (FA) on dogs. J Toxicol Sci. 2004;29(2):155-166.)

Establishing that the preferential cytotoxicity of F10 towards malignant cells *in vitro* can be achieved *in vivo* is a remarkable accomplishment that establishes the feasibility of using F10 for treatment of GBM in humans, particularly with local administration. To date, conventional FPs have displayed limited utility for GBM treatment in humans. (Miller et al., Intratumoral 5-fluorouracil produced by cytosine deaminase/5-fluorocytosine gene therapy is effective for experimental human glioblastomas. Cancer Res. 2002;62(3):773-780.) Thus, the promising activity towards GBM observed in the present studies with F10 indicates this novel polymeric FP may be fundamentally different from other FPs in this regard.

The mechanistic basis for the selective cytotoxicity of F10 towards malignant cells likely is multi-faceted. Previous studies demonstrated that F10 is cytotoxic to AML cells at nM concentrations through dual targeting of TS and Top1, two of the best validated targets for anti-cancer drugs. (Pardee et al., Unique dual targeting of thymidylate synthase and topoisomerase 1 by FdUMP[10] results in high efficacy against AML and low toxicity. Blood. 2012; 119(15):3561-3570.) While certain GBM cells are also sensitive to F10 at nM concentrations (e.g. SF268) the G48a cells as well as U-251 MG and SNB-19 cells used for the present studies are somewhat less sensitive. This reduced sensitivity of F10 for certain GBM cells (relative to AML cells) correlates with higher concentrations of F10 being required for TS inhibition in these cells **(****Fig. 1D****)** and inversely correlates with TS expression levels **(****Fig. 1E****).** As F10 cytotoxicity results from incorporation of FdUTP into DNA, strategies to reduce TS expression or reduce TS activity in GBM cells and thus create a thymineless state are expected to enhance F10 activity for GBM treatment. In this regard, recent studies implicating EGFR expression and activity as modulating TS expression provide a basis for exploring the combined effects of F10 with EGFR inhibitors for treatment of GBM, a malignancy frequently characterized by elevated EGFR.

In summary, F10 displays strong anti-tumor activity towards an orthotopic GBM tumor in nude mice upon i.c. administration. F10 is not cytotoxic towards primary neuronal cultures *in vitro,* and strong anti-tumor activity was achieved without apparent damage to normal brain tissue *in vivo.* This demonstrates that F10 will prove useful for improving survival outcomes for GBM patients.

### EXAMPLE 2: Combination Treatments

The anti-tumor and radiosensitizing activities of FdUMP[10] in combination with Chk1/2 inhibitors are evaluated using an orthotopic xenograft model of GBM. FdUMP[10]/TMZ with and without radiation is also evaluated in these studies since any alternative combination involving FdUMP[10] (*e.g.*, FdUMP[10]/AZD7762) is expected to display a superior efficacy/toxicity profile to warrant clinical translation.

Data indicate FdUMP[10] is efficacious at much lower doses administered locally (e.g. 120 mg/kg over 7 days for orthotopic GBM vs. 300 mg/kg qodx4 for systemic AML treatment) strongly suggesting limited permeability of the BBB. It is therefore expected that the highest levels of 14C retention in brain tumor tissue will occur with i.c. administration of FdUMP[10].

**Combined treatment with FdUMP[10] and AZD7762 led to a decreased clonogenic survival of U138 cells (****FIG. 7****).** 500-2000 U138 cells were seeded into 60mm dishes and treated with 1nM FdUMP[10] (F1) and/or 10 nM AZD7726 (A2) and the drugs were removed after 72 h. Cells were fixed and stained with violet blue and colonies were counted using a colony counter 10 days post-treatment. FdUMP[10] and AZD7762 treatment resulted in 0.84 and 0.80 survival fractions, respectively. FdUMP[10]/AZD7762 co-treatment resulted in a 0.62 survival fraction exceeding the 0.67 expected based on an additive interaction. The results are consistent with FdUMP[10]AZD7762 being synergistic at decreasing the clonogenicity of U138 cells.

**Treating U138 cells with FdUMP[10]/AZD7762 + IR results in predominantly apoptotic cell death (****FIG. 8****).** U138 cells were treated with 1nM FdUMP[10] and/or 10 nM of AZD7762 for 9 or 1 h, respectively, prior to irradiation with a single dose of 6 Gy. Cells were harvested 24 h post-irradiation and analyzed for DNA content (A) and cleaved caspase 3 (B) by flow cytometry. (A) DNA histograms showing FdUMP[10] causes G2-arrest. Co-treatment with AZD7762 abrogates G2-arrest and results in cells with sub-G0 DNA content. Irradiation enhances the percentage of cells with sub- G0 content for FdUMP[10] and FdUMP[10]/AZD7762 treatments. (B) Cleaved caspase 3 (x-axis) vs DNA content (y-axis). Vehicle-only (not shown), IR-only, AZD7762-only (not shown) and AZD7762 + IR do not result in significant percent of cleaved caspase 3 while FdUMP[10]+IR and FdUMP[10]/AZD7762+IR treatments result predominantly in apoptotic cells.

**FdUMP[10]-Induced Cell-Cycle Arrest and Activation of HRR:** FdUMP[10] is highly cytotoxic towards GBM cells however curing GBM, or providing a long-term survival advantage, requires essentially total tumor eradication. With CED and retention within the BBB, it is expected to achieve high local concentrations of FdUMP[10] that have the potential for complete tumor eradication.

**Synergy of FdUMP[10]/AZD7762 Towards GBM Cells:** It was found that the combination of FdUMP[10] + the Chk1/2 inhibitor AZD7762 is synergistic at decreasing the clonogenic survival of GBM cells **(****Figure 7****).** Data also indicate that FdUMP[10] (1 nM; 8 h) followed by AZD7762 (10 nM; 1 h) is mildly synergistic.

**Radiosensitization of FdUMP[10]/AZD7762.** Radiation is cytotoxic as a consequence of unrepaired DNA damage. FdUMP[10]/AZD7762 is radiosensitizing while neither FdUMP[10] nor AZD7762 are appreciably radiosensitizing (e.g. additive effects) as single-agents. In these studies, FdUMP[10] was administered 6 h prior to irradiation and AZD7762 1 h prior to radiation. Though not wishing to be bound by theory, it is thought that radiosensitization results from induction of a thymineless state following FdUMP[10] treatment and disabling of HRR and abrogation of the G2 checkpoint following AZD7762 treatment. Under these conditions, DNA repair of radiation-induced DNA damage is inhibited due to lack of thymidine while inhibiting Rad51 activation prevents HRR which is important for repair of radiation-induced DNA DSBs (Helleday 2010). Abrogating the G2-checkpoint causes cells to enter mitosis with lethally-damaged DNA enhancing the cytotoxic effects of radiation.

Studies demonstrate that co-treatment of U138 GBM cells with FdUMP[10] and the Chk1/2 inhibitor AZD7762 is synergistic with respect to clonogenic survival and induction of apoptosis **(****FIG. 7 and FIG. 8****).** Importantly, FdUMP[10]/AZD7762 is radiosensitizing **(****FIG. 9****).**

The foregoing is illustrative of the present invention, and is not to be construed as limiting thereof. The invention is defined by the following claims, with equivalents of the claims to be included therein.

## Claims

1. Poly-FdUMP or a pharmaceutically acceptable salt thereof for use in treating a brain tumor in a subject.

2. The poly-FdUMP or a pharmaceutically acceptable salt thereof of claim 1 for use of claim 1, wherein said subject is a human subject.

3. The poly-FdUMP or a pharmaceutically acceptable salt thereof of claim 1 or 2 for use of claim 1 or 2, wherein said brain tumor is selected from the group consisting of:
(i) a glioma;
(ii) an astrocytoma; and
(iii) glioblastoma multiforme (GBM).

4. The poly-FdUMP or a pharmaceutically acceptable salt thereof of claim 1 to 3 for use of claim 1 to 3, wherein said active agent is intended to be administered to said subject by one or more methods selected from the group consisting of:
(i) intra-cerebral administration;
(ii) intracerebroventricular infusion; and
(iii) intrathecal infusion.

5. The poly-FdUMP or a pharmaceutically acceptable salt thereof of any one of claims 1 to 3 for use of any one of claims 1 to 3, wherein said active agent is intended to be administered into the brain of said subject by convection-enhanced delivery (CED).

6. The poly-FdUMP or a pharmaceutically acceptable salt thereof of any one of claims 1 to 3 for use of any one of claims 1 to 3, wherein said active agent is intended to be administered as a liposomal formulation.

7. The poly-FdUMP or a pharmaceutically acceptable salt thereof of any one of claims 1 to 6 for use of any one of claims 1 to 6, wherein said subject is at least 60, 65 or 70 years old.

8. The poly-FdUMP or a pharmaceutically acceptable salt thereof of any one of claims 1 to 7 for use of any one of claims 1 to 7, wherein said active agent is intended to be administered in an amount of from 50 to 400 mg/kg and/or by continuous infusion over a period of from 7 to 14 days.

9. The poly-FdUMP or a pharmaceutically acceptable salt thereof of any one of claims 1 to 8 for use of any one of claims 1 to 8, wherein said poly-FdUMP is FdUMP[9] or FdUMP[10].

10. The poly-FdUMP or a pharmaceutically acceptable salt thereof of any one of claims 1 to 9 for use of any one of claims 1 to 9, wherein said poly-FdUMP is covalently linked at the 3' end thereof to a levulinyl group or a hydrophobic molecule.

11. The poly-FdUMP or a pharmaceutically acceptable salt thereof of claim 10 for use of claim 10, wherein said hydrophobic molecule comprises cholesterol.

12. The poly-FdUMP or a pharmaceutically acceptable salt thereof of any one of claims 1 to 11 for use of any one of claims 1 to 11, wherein said active agent is intended to be administered in combination with one or more therapies selected from: another thymidylate synthase (TS) inhibitor, an epidermal growth factor receptor (EGFR) inhibitor, an inhibitor of casein kinase 1 or casein kinase 2, temozolomide (TMZ), and radiation.

13. A composition comprising:
a) a poly-FdUMP; and
b) an active agent selected from: another thymidylate synthase (TS) inhibitor, an epidermal growth factor receptor (EGFR) inhibitor, an inhibitor of casein kinase 1 and/or casein kinase 2, temozolomide (TMZ), and a combination thereof.

14. The composition of claim 13, wherein said composition further comprises a pharmaceutically acceptable carrier, an aqueous carrier or a carrier comprising saline.

15. The composition of claim 13, wherein said active agent is another thymidylate synthase inhibitor comprising a nucleic acid, and said poly-FdUMP is coupled to said thymidylate synthase inhibitor; or wherein said active agent is an EGFR ligand, and said poly-FdUMP is coupled to said EGFR ligand.
